# EUROPÄISCHE PATENTSCHRIFT

(11) **EP 1 913 146 B1**
(45) Veröffentlichungstag und Bekanntmachung des Hinweises auf die Patenterteilung: **26.10.2011**
(21) Anmeldenummer: 06792615.4
(22) Anmeldetag: 31.07.2006
(51) Int. Cl.: C12P 7/62

(54) **Enzymatische Herstellung von (Meth)acrylsäureestern**
Enzymatic production of (meth)acrylic acid esters
Production enzymatique d'esters d'acide (meth)acrylique

(30) Priorität: 04.08.2005 DE 102005037430
(43) Veröffentlichungstag der Anmeldung: 23.04.2008
(73) Patentinhaber: BASF SE, 67056 Ludwigshafen (DE)
(72) Erfinder: HÄRING, Dietmar, 68535 Neu-Edingen (DE); MEISENBURG, Uwe, 68161 Mannheim (DE); HAUER, Bernhard, 67136 Fussgönheim (DE); DIETSCHE, Frank, 69198 Schriesheim (DE)
(86) Internationale Anmeldenummer: PCT/EP2006/064848
(87) Internationale Veröffentlichungsnummer: WO 2007/014935

(56) Entgegenhaltungen:
- EP-A- 0 457 332
- EP-A- 0 508 491
- EP-A- 0 566 095
- WO-A-2005/059151
- GB-A- 465 789
- US-A1- 2002 102 671
- DATABASE WPI Week 197124 1971, Derwent Publications Ltd., London, GB; AN 1971-41092S XP002403470 & SU 276 046 A (ABDULIAEV SH U BONDARYUK) 14. Dezember 1970 (1970-12-14) -& SU 276 046 A 14. Dezember 1970 (1970-12-14)
- DATABASE CAPLUS CHEMICAL ABSTRACTS SERVICE, COLUMBUS, OHIO, US; 1986, GILAZHOV, E.G.: "Synthesis of unsaturated esters of cyclic and heterocyclic acetylenic alcohols and their linear polymers" XP002401968 gefunden im STN accession no. 1986:19850 Database accession no. 104:19850
- BURGESS K ET AL: "BIOCATALYTIC RESOLUTIONS OF ALPHA METHYLENE-BETA-HYDROXY ESTERS AND KETONES" JOURNAL OF ORGANIC CHEMISTRY, Bd. 55, Nr. 4, 1990, Seiten 1138-1139, XP002401966 ISSN: 0022-3263
- WALDINGER C ET AL: "Aryl Propargylic Alcohols Of High Enantiomeric Purity via Lipase Catalyzed Resolutions" TETRAHEDRON: ASYMMETRY, ELSEVIER SCIENCE PUBLISHERS, AMSTERDAM, NL, Bd. 7, Nr. 5, Mai 1996 (1996-05), Seiten 1485-1488, XP004047709 ISSN: 0957-4166
- LIE MARCEL S F ET AL: "Lipase specificity toward some acetylenic and olefinic alcohols in the esterification of pentanoic and stearic acids" LIPIDS, Bd. 33, Nr. 9, September 1998 (1998-09), Seiten 861-867, XP008069811 ISSN: 0024-4201
- WARWEL SIEGFRIED ET AL: "An efficient method for lipase-catalysed preparation of acrylic and methacrylic acid esters" BIOTECHNOLOGY TECHNIQUES, Bd. 10, Nr. 4, 1996, Seiten 283-286, XP008069801 ISSN: 0951-208X

## Beschreibung

Die vorliegende Erfindung betrifft ein Verfahren zur Herstellung von (Meth)acrylsäureestern von Alkoholen mit C-C-Dreifachbindungen ausgewählt aus Propargylalkohol oder dessen ein- oder zweifach ethoxylierter Produkte sowie Gemische daraus, Verfahren zu deren Herstellung und deren Verwendung.

Die Herstellung von (Meth)acrylsäureestern erfolgt zumeist durch säure- oder basenkatalysierte Veresterung von (Meth)acrylsäure oder Umesterung von anderen (Meth)-acrylsäureestern mit Alkoholen oder durch Reaktion von Alkoholen mit (Meth)acryloylchlorid in Gegenwart von Basen.

(Meth)acrylsäureester von Alkoholen mit C-C-Dreifachbindungen sind prinzipiell bekannt. Bei der klassischen Veresterung von derartigen Alkoholen mit z.B. Säuren werden jedoch durch säureinduzierte Nebenreaktionen, insbesondere Isomerisierungen der Dreifachbindung meist uneinheitliche, zumindest aber stark gefärbte Produktgemische erhalten.

Ähnlich wie bei der Wanderung von C-C-Doppelbindungen durch Allyl-Umlagerung kann in Alkinen die C-C-Dreifachbindung wandern (Alkin-Allen-Umlagerung). Derartige Isomerisierungen sind oft Gleichgewichtsreaktionen. Die Umlagerung erfolgt beispielsweise durch Wanderung eines Anions oder Kations und wird durch Säuren oder Basen katalysiert (Houben-Weyl, Methoden der organischen Chemie Band V/2a, Thieme Verlag 1977, Seite 282 ff). Durch diese Instabilität der Dreifachbindung neigen Alkine unter sauren oder basischen Bedingungen zur Bildung von Nebenprodukten (z.B. Allene und deren Folgeprodukte), die aus dem Reaktionsgemisch aufwendig destillativ oder durch Extraktion entfernt werden müssen.

In EP 508491 (Wacker Chemie, 1992) wurde 2-Propinoxyethanol mit Acrylsäure in Toluol als Schleppmittel azeotrop verestert unter Katalyse von Schwefelsäure. Nach 4 h bei 120 °C wurde die überschüssige Acrylsäure mit NaHCO₃ neutralisiert und mit Wasser ausgewaschen. Anschließend wurde Toluol abdestilliert und 2-Propargyloxethylacrylat destillativ gereinigt. Man erhielt mit 85 % Ausbeute ein leicht gelbliches Produkt.

Bei basenkatalysierter Umesterung oder anderen Synthesen, z.B. mit Metallkomplexen, entstehen aus dem gleichen Grunde ebenfalls komplexe und gefärbte Produktgemische. Zur Entfernung von Färbungen und unumgesetzten Reaktanden müssen die Produktgemische durch aufwendige alkalische Wäschen aufgearbeitet werden.

Die Herstellung aus Acryloylchlorid in Gegenwart von Basen ist ebenfalls bekannt:

In US 3254115 (Thiokol Chemical Corp., 1966) wird die Herstellung von Propargyl-Acrylat aus Propargylalkohol und Acryloylchlorid beschrieben. Katalysator ist Triethylamin und als Lösungsmittel wird Benzol verwendet. Das Produkt wurde im Vakuum destilliert mit 60 % Ausbeute.

Harvey et al. (J. Am. Chem. Soc., 1992, 114, 8424-8434) stellen Propargyl-Acrylat ebenfalls aus Propargylalkohol und Acryloylchlorid her. Katalysator ist Triethylamin und als Lösungsmittel wird Dichlormethan verwendet. Das Produkt wird nacheinander mehrfach mit HCl, NaHCO₃ und Wasser extrahiert mit 90 % Ausbeute.

Die Herstellung von (Meth)acrylsäureestern durch eine enzymatische Ver- oder Umesterung ist bekannt.

Kumar und Gross beschreiben in J. Am. Chem. Soc. 2002, 124, 1850-1851 die Lipase-katalysierte Umsetzung von Isopropyliden-geschützten Zuckern durch Umsetzung mit Vinylmethacrylat. Eine vollständige Umsetzung wird erreicht durch das spezielle Edukt Vinylmethacrylat, da freigesetzter Vinylalkohol dem Reaktionsgleichgewicht als Acetaldehyd entzogen wird. Nachteilig an diesem Verfahren ist, daß Vinylmethacrylat als Spezialmonomer teuer und kommerziell nur in kleinen Mengen verfügbar ist.

A. T. J. W. de Goede et al. beschreiben in Biocatalysis, 1994, 9, 145-155 die Umesterung von α-O-Octyl-Glucosid mit Ethylacrylat zum 6-O-Acrylester in Gegenwart von Lipasen. Nachteilig an diesem Verfahren ist, daß es auf Glucoside und glykosidische Bindungen beschränkt ist und empfindlich auf sterische Einflüsse im Glucosid reagiert. Zudem wurden höher acrylierte Produkte infolge von unselektiven Nebenreaktionen erhalten.

EP-A1 999 229 beschreibt die enzymatische Ver- und Umesterung von Polyoxyalkylenen mit (Meth)Acrylsäure und (Meth)Acrylsäureestern.

Aus WO 03/042227 ist die Lipase-katalysierte Umesterung von Alkylacrylaten mit Zuckern bekannt.

US 5240835 beschreibt die Umesterung von Alkylacrylaten mit Alkoholen unter Katalyse eines Biokatalysators aus Corynebacterium oxydans. Beispielhaft wird dort die Reaktion von einem 96-fachen molaren Überschuß Ethylacrylat mit 2,2-Dimethyl-1,3-propandiol aufgeführt. Lediglich 21 % Ausbeute wurden nach 3 Tagen bei 30 °C erhalten.

Athawale und Manjrekar (Tetrahedron Lett. 2001, 42 4541-4543) beschreiben die Lipase-katalysierte Acrylierung von Alkaloiden mittels 2,3-Butandion-monooximacrylat. Das Monomer wurde polymerisiert und zur Induktion von enantioselektiven Michael-Addition eingesetzt.

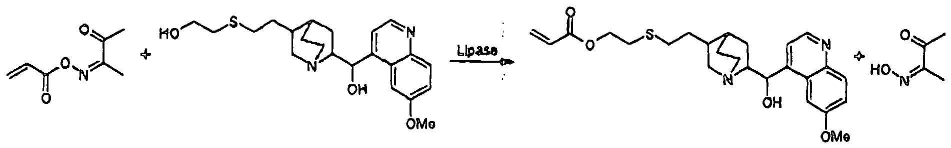

Athawale und Gaonkar (Makromolecules 1999,32, 6065-6068) beschreiben die Lipase-katalysierte Acrylierung von 2-Phenylethanolen mittels 2,3-Butandion-monooxim-acrylat. Das Monomer wurde anschließend polymerisiert.

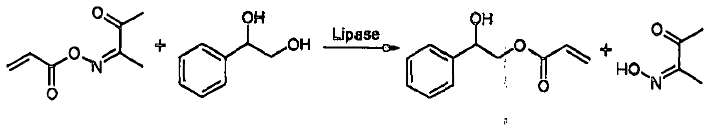

Ghogare und Kumar (J. Chem. Soc. 1989, 1533-1535) beschreiben die Lipase-kataly-sierte Acrylierung von verschiedenen Alkoholen, u.a. 2-Ethylhexan-1,3-diol, mit einem aktivierten 2,3-Butandion-monooximacrylat.

Nachteilig an diesen Reaktionen ist häufig die Notwendigkeit zur Verwendung von aktivierten Acrylaten, wie z.B. die Oxime oder Vinylester, die teuer und industriell schlecht verfügbar sind.

Weiterhin ist aus SU 276 046 ein Verfahren zur Herstellung von Propargylestern der (Meth)acrylsäure bekannt. Demnach erfolgt die Umesterung des entsprechenden Methylesters mit Propargylalkohol in Gegenwart von Na- oder Ti- alkoxiden als bevorzugte Katalysatoren.

GB 465,789 offenbart ebenfalls ein Verfahren zur Herstellung von Methacrylsäureestern, die durch Umsetzung von Methacrylsäure mit einem ungesättigten Alkohol erhalten werden.

Aus Database Caplus, Chemical Abstracts Service, Columbus, Ohio, US; 1986, Gilazhov, E.G. STN accession no. 1986 : 19850, Database accession no. 104 : 19850 ist die Synthese von ungesättigten Estern von cyclischen und heterocyclischen acetylenischen Alkoholen bekannt.

EP 0 457 332 A1 offenbart ethylenische und acetylenische Gruppen enthaltende polymerisierbare Carbonate, die ausgehend von Propargylalkohol bzw. dessen Chlorformiats erhalten werden.

Aus EP 0 566 095 A1 ist ebenfalls die Verwendung von Propargylalkohol bekannt. Demnach wird eine härtbare Harzzusammensetzung beschrieben, die ein Polymer mit Alkenylgruppen, ein Organowasserstoffpolysiloxan, ein Polymer mit Alkinylgruppen und einen Hydrosilylierungskatalysator umfasst.

Die enzymkatalysierte Herstellung von (Alk)acrylaten durch Ver- oder Umesterung ausgehend von Arylketonen ist aus WO 2005/059151 A2 bekannt.

Aufgabe der vorliegenden Erfindung war es, ein Verfahren zur Verfügung zu stellen, mit dem (Meth)acrylsäureester von Alkoholen, die C-C-Dreifachbindungen aufweisen und die ausgewählt sind aus Propargylalkohol oder dessen ein- oder zweifach ethoxylierter Produkte sowie Gemische daraus, in guter Ausbeute und niedrigen Farbzahlen hergestellt aus einfachen Edukten werden können. Die Synthese sollte unter milden Bedingungen ablaufen, so daß Produkte mit einer niedrigen Farbzahl und hoher Reinheit resultieren. Speziell auf den Einsatz von sauren oder basischen Katalysatoren sollte verzichtet werden, um die Entstehung von Nebenprodukten zu verhindern. Dadurch kann auf eine aufwendige destillative oder extraktive Produktreinigung verzichtet werden. Zudem soll auf den Einsatz teurer, aktivierter (Meth)Acrylsäurederivate, wie beispielsweise Monooxime oder Vinyl(meth)-acrylat verzichtet werden.

Die Aufgabe wurde gelöst durch ein Verfahren zur Herstellung von (Meth)acrylsäureestern (F) von mindestens eine C-C-Dreifachbindung aufweisenden Alkoholen, in dem man mindestens einen mindestens eine C-C-Dreifachbindung aufweisenden Alkohol ausgewählt aus Propargylalkohol oder dessen ein- oder zweifach ethoxylierter Produkte sowie Gemische daraus,

in Gegenwart mindestens eines Enzyms (E) mit mindestens einem (Meth)acrylsäureester (D) umestert,
wobei der (Meth)acrylsäureester (D) ein gesättigter C₁ - C₁₀-Alkylester oder C₃ - C₁₂-Cycloalkylester der (Meth)acrylsäure ist,
wobei das Enzym (E) eine Lipase aus Candida antarctica B ist, und
wobei das molare Verhältnis von (Meth)acrylsäureverbindung (D) (bezogen auf die (Meth)acryleinheiten) zu Alkohol (C) (bezogen auf Hydroxygruppen) im Bereich von 20:1 bis 2:1 liegt.

Mit Hilfe des erfindungsgemäßen Verfahrens ist die Herstellung derartiger (Meth)acrylsäureester (F) in hoher chemischer und Raum-Zeit-Ausbeute und unter milden Bedingungen mit guten Farbzahlen unter Verzicht auf Schutzgruppenoperationen und unter Einsatz einfacher Ausgangsstoffe möglich.

(Meth)acrylsäure steht in dieser Schrift für Methacrylsäure und Acrylsäure, bevorzugt für Acrylsäure.

Erfindungsgemäß geeignete Alkohole (C) sind solche Alkohole, die mindestens eine C-C- Dreifachbindung und mindestens eine Hydroxygruppe enthalten, und die ausgewählt sind aus Propargylalkohol oder dessen ein- oder zweifach ethoxylierter Produkte sowie Gemische daraus.

Im Reaktionsschritt erfolgt die Umesterung des Alkohols (C) mit mindestens einem, bevorzugt einem (Meth)acrylat (D) in Anwesenheit mindestens eines, bevorzugt eines die Umesterung katalysierenden Enzyms (E).

Verbindungen (D) können Ester von (Meth)acrylsäure mit einem gesättigten Alkohol sein, bevorzugt gesättigte C₁ - C₁₀-Alkylester oder C₃ - C₁₂-Cyclo-alkylester der (Meth)acrylsäure, besonders bevorzugt gesättigte C₁ - C₄-Alkylester der (Meth)acrylsäure.

Gesättigt bedeutet im Rahmen dieser Schrift Verbindungen ohne C-C-Mehrfach-bindungen (außer selbstverständlich die C=C-Doppelbindung in den (Meth)acryl-einheiten).

Beispiele für Verbindungen (D) sind (Meth)acrylsäuremethyl-, -ethyl-, -n-butyl-, -*iso*-butyl-, n-octyl- und -2-Ethylhexylester, 1,2-Ethylenglycoldi- und -mono(meth)-acrylat, 1,4-Butandioldi- und -mono(meth)acrylat, 1,6-Hexandioldi- und -mono(meth)-acrylat, Trimethylolpropantri(meth)acrylat und Pentaerythrittetra(meth)acrylat.

Besonders bevorzugt sind (Meth)acrylsäuremethyl-, -ethyl-, -n-butyl- und -2-Ethyl-hexylester und ganz besonders bevorzugt (Meth)acrylsäuremethyl-, -ethyl- und -n-butylester,

Soweit die genannten Alkohole optisch aktiv sind werden sie bevorzugt racemisch oder als Diastereomerengemische eingesetzt, es ist jedoch auch möglich, sie als reine Enantiomere bzw. Diastereomere oder als Enantiomerengemische einzusetzen.

Die enzymatische Ver- oder Umesterung mit einem (Meth)acrylat erfolgt im Allgemeinen bei 0 bis 100°C, bevorzugt 20 bis 80 °C, besonders bevorzugt 20 bis 70°C, ganz besonders bevorzugt 20 bis 60 °C.

Das erfindungsgemäß einsetzbare Enzym (E) wird in freier oder auf einem Träger chemisch oder physikalisch immobilisierter Form eingesetzt. Erfindungsgemäß wird als Enzym (E) eine Lipase B aus *Candida antarctica* verwendet.

Der Enzymgehalt im Reaktionsmedium liegt in der Regel im Bereich von etwa 0,1 bis 10 Gew.-%, bezogen auf den eingesetzten Alkohol (C).

Die Reaktionszeit hängt unter anderem von der Temperatur, der verwendeten Menge und der Aktivität des Enzymkatalysators und vom geforderten Umsatz ab sowie vom Alkohol. Bevorzugt wird die Reaktionszeit so angepaßt, daß der Umsatz der umzusetzenden im Alkohol (C) enthaltenden, d.h. der niedrigersubstituierten Hydroxyfunktionen mindestens 70%, bevorzugt mindestens 80, besonders bevorzugt mindestens 90, ganz besonders bevorzugt mindestens 95 %, insbesondere mindestens 97% und speziell mindestens 98 % beträgt. In der Regel sind dafür 1 bis 72 Stunden, bevorzugt 3 bis 36 und besonders bevorzugt 3 bis 24 Stunden ausreichend.

Das molare Verhältnis von (Meth)acrylsäureverbindung (D) (bezogen auf die (Meth)-acryleinheiten) zu Alkohol (C) (bezogen auf Hydroxygruppen) liegt erfindungsgemäß im Bereich von 20:1 bis 2:1 und bevorzugt von 20:1 bis 4:1

Die Reaktion kann in organischen Lösungsmitteln oder deren Gemischen oder ohne Zusatz von Lösungsmitteln ablaufen. Bevorzugt wird kein Lösungsmittel zugesetzt. Die Ansätze sind in der Regel weitgehend wasserfrei (d.h. unter 10, bevorzugt unter 5, besonders bevorzugt unter 1 und ganz besonders bevorzugt unter 0,5 Vol% Wasserzusatz).

Geeignete organische Lösungsmittel sind solche für diese Zwecke bekannten, beispielsweise tertiäre Monoole, wie C₃-C₆-Alkohole, bevorzugt tert-Butanol, tert-Amyl-alkohol, Pyridin, Poly-C₁-C₄--alkylenglykoldi-C₁-C₄-Alkylether, bevorzugt Polyethylen-glycoldi-C₁-C₄-alkylether, wie z.B. 1,2-Dimethoxyethan, Diethylenglycoldimethylether, Polyethylenglycoldimethylether 500, Methyl-tert.Butylether, Ethyl-tert.Butylether,
C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, *iso*-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen. Es kann vorteilhaft sein, freiwerdendes Wasser oder Alkohol durch ein möglichst nahe am Temperaturoptimum des verwendeten Enzyms siedendes binäres oder ternäres Heteroazeotrop abzutrennen. Der so entfernte Alkohol kann dann durch Phasenscheidung oder Membrandampftrennung entfernt werden.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z.B. 10 bis 100mM) Puffer, beispielsweise mit einem pH-Wert im Bereich von etwa 6 bis 8, wie z.B. Kaliumphosphat- oder TRIS-HCl-Puffer.

Der Wasseranteil im Reaktionsansatz liegt in der Regel bei 0-10 Vol%. Bevorzugt werden die Reaktanden ohne Vorbehandlung (Trocknung, Wasserdotierung) eingesetzt.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol gleichzeitig abdestilliert werden kann.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z.B. durch Vakuum, azeotrope Entfernung, Absorption, Pervaporation und Diffusion über Membranen.

Hierzu eignen sich vorzugsweise Molekularsiebe oder Zeolithe (Porengröße z.B. im Bereich von etwa 3-10 Angström), eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wiederzuverwerten.

Nach Beendigung der Reaktion kann man das aus der Umesterung erhaltene Reaktionsgemisch ohne weitere Aufreinigung weiterverwenden oder es erforderlichenfalls in einem weiteren Schritt aufreinigen.

In der Regel wird in einem Reinigungsschritt lediglich das eingesetzten Enzym vom Reaktionsgemisch abgetrennt und das Reaktionsprodukt vom gegebenenfalls verwendeten organischen Lösungsmittel abgetrennt.

Eine Abtrennung vom Enzym erfolgt in der Regel durch Filtration, Absorption, Zentrifugation oder Dekantieren. Das abgetrennte Enzym kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden im Reinigungsschritt jedoch lediglich das eingesetzte Enzym und das gegebenenfalls eingesetzte Lösungsmittel oder der Überschuß (Meth)Acrylat abgetrennt.

Die Reaktionsbedingungen bei der enzymatischen Umesterung sind mild. Aufgrund der niedrigen Temperaturen und sonstigen milden Bedingungen wird die Bildung von Nebenprodukten während der Reaktion vermieden, die andernfalls zum Beispiel von chemischen Katalysatoren stammen können oder durch unerwünschte radikalische Polymerisation des eingesetzten (Meth)acrylats, die sonst nur durch Zugabe von Stabilisatoren verhindert werden kann.

Bei der erfindungsgemäßen Reaktionsführung kann der (Meth)acrylverbindung (D) über den ohnehin enthaltenen Lagerstabilisator hinaus zusätzliche Stabilisator zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethyl-piperi-din-N-oxyl, beispielsweise in Mengen von 50 bis 2000 ppm. Vorteilhaft wird die Ver- oder Umesterung in Gegenwart eines sauerstoffhaltigen Gases, bevorzugt Luft oder Luft-Stickstoff-Gemische, durchgeführt.

Des weiteren kann der Enzymkatalysator unproblematisch vom Endprodukt entfernt werden.

Das Reaktionsgemisches kann gegebenenfalls falls gewünscht aufgereinigt werden, beispielsweise durch Filtration, Destillation, Rektifikation, Chromatographie, Behandlung mit Ionentauschern, Adsorbentien, neutraler, saurer und/oder alkalischer Wäsche, Strippen oder Kristallisation.

Ein weiterer Gegenstand der vorliegenden Anmeldung sind die aus den Alkoholen (C) durch enzymatische Umesterung erhaltenen (Meth)acrylate. Diese weisen durch die erfindungsgemäßen Reaktionsbedingungen eine Farbzahl unter 100 APHA gemäß DIN ISO 6271, bevorzugt unter 80. Ferner enthalten sie in der Regel weniger als 1,0 % Nebenprodukte aus Umlagerungsreaktionen der Mehrfachbindung aus säure- oder basenkatalysierten Nebenreaktionen.

Der Vorteil der so nach dem erfindungsgemäßen Verfahren erhaltenen (Meth)Acrylsäureester ist der, daß sie wegen ihrer geringen Farbzahl in Lackanwendungen und dort insbesondere in Klarlacken vorteilhaft eingesetzt werden können, da sie durch ihre geringe Eigenfärbung eine verringerte Färbung der Beschichtungen gegenüber nach konventionellen Verfahren hergestellten Alkoholen bewirken.

Ferner können Beschichtungen mit den erfindungsgemäß hergestellten Estern sehr hohe Kratzfestigkeiten, Härten, Chemikalienbeständigkeiten, Elastizität und Haftung, sowohl auf hydrophilen als auch auf hydrophoben Substraten aufweisen.

Die erfindungsgemäß erhältlichen (Meth)acrylsäureester (F) können vorteilhaft als Monomere oder Comonomere in Poly(meth)acrylaten oder als Reaktivverdünner in thermisch-, strahlungs- und/oder Dual-Cure-härtbaren Poly(meth)acrylaten eingesetzt werden. Derartige Poly(meth)acrylate sind beispielsweise als Bindemittel in thermisch-, strahlungs- oder Dual-Cure-härtbaren Beschichtungsmitteln sowie in Klebstoffen, z.B. Acrylatklebstoffen geeignet sowie in Dichtungsmassen.

Ein weiterer Gegenstand der vorliegenden Anmeldung ist daher die Verwendung der nach dem erfindungsgemäßen Verfahren hergestellten (Meth)acrylsäureester als Reaktivverdünner oder Bindemittel in Strahlen oder Dual-Cure-härtbaren Beschichtungsmassen, bevorzugt in Deckbeschichtungen, besonders bevorzugt in transparenten Klarlacken. Selbstverständlich können die erfindungsgemäß hergestellten (Meth)acryl-säureester auch als Monomere in Polymerisationen, gegebenenfalls zusammen mit anderen polymerisierbaren Monomeren, wie z.B. (Meth)acrylsäure, (Meth)acryl-säureester, Styrol, Butadien, Acrylnitril, Vinylacetat, N-Vinylpyrrolidon, 4-Hydroxybutyl-vinylether oder N-Vinylformamid, verwendet werden.

Unter "Dual-Cure" ist zu verstehen, dass die Beschichtungsmassen thermisch und mit aktinischer Strahlung härtbar sind. Im Rahmen der vorliegenden Erfindung ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen.

Strahlenhärtbare Bindemittel sind solche, die mittels aktinischer Strahlung wie vorstehend definiert, insbesondere mittels UV-Strahlung härtbar sind.

Ein weiterer Gegenstand der vorliegenden Anmeldung sind Lackformulierungen, enthaltend die nach dem erfindungsgemäßen Verfahren erhältlichen (Meth)acrylsäureester. Dabei können die (Meth)acrylsäureester sowohl in Basislacken als auch in Decklacken eingesetzt werden. Aufgrund ihrer besonderen Eigenschaften, insbesondere ihrer geringen Farbzahl, ist ihr Einsatz in Deckbeschichtungen und einer strahlengehärteten Klarlackbeschichtungen bevorzugt.

Neben den nach dem erfindungsgemäßen Verfahren erhältlichen (Meth)acrylsäureester (F) kann eine strahlungshärtbare Masse noch folgende Komponenten enthalten:
(G) mindestens eine polymerisierbare Verbindung mit mehreren copolymerisierbaren, ethylenisch ungesättigten Gruppen,
(H) gegebenenfalls Reaktiwerdünner,
(I) gegebenenfalls Photoinitiator sowie
(J) gegebenenfalls weitere lacktypische Additive.

Als Verbindungen (G) kommen strahlungshärtbare, radikalisch polymerisierbare Verbindungen mit mehreren, d.h. mindestens zwei, copolymerisierbaren, ethylenisch ungesättigten Gruppen in Betracht.

Bevorzugt handelt es sich bei Verbindungen (G) um Vinylether- oder (Meth)acrylatverbindungen, besonders bevorzugt sind jeweils die Acrylatverbindungen, d.h. die Derivate der Acrylsäure.

Bevorzugte Vinylether- und (Meth)acrylat-Verbindungen (G) enthalten 2 bis 20, bevorzugt 2 bis 10 und ganz besonders bevorzugt 2 bis 6 copolymerisierbare, ethylenisch ungesättigte Doppelbindungen.

Besonders bevorzugt sind solche Verbindungen (G) mit einem Gehalt an ethylenisch ungesättigte Doppelbindungen von 0,1 - 0,7 mol /100 g, ganz besonders bevorzugt 0,2 - 0,6 mol / 100 g.

Das zahlenmittlere Molekulargewicht Mₙ der Verbindungen (G) liegt, wenn nicht anders angegeben, bevorzugt unter 15000, besonders bevorzugt bei 300 - 12000, ganz besonders bevorzugt bei 400 bis 5000 und insbesondere bei 500 - 3000 g/mol (bestimmt durch Gelpermeationschromatographie mit Polystyrol als Standard und Tetrahydrofuran als Elutionsmittel).

Als (Meth)acrylatverbindungen genannt seien (Meth)acrylsäureester und insbesondere Acrylsäureester sowie Vinylether von mehrfunktionellen Alkoholen, insbesondere solchen, die neben den Hydroxylgruppen keine weiteren funktionellen Gruppen oder allenfalls Ethergruppen enthalten. Beispiele solcher Alkohole sind z.B. bifunktionelle Alkohole, wie Ethylenglykol, Propylenglykol und deren höher kondensierte Vertreter, z.B. wie Diethylenglykol, Triethylenglykol, Dipropylenglykol, Tripropylenglykol etc., 1,2-, 1,3- oder 1,4-Butandiol, 1,5-Pentandiol, 1,6-Hexandiol, 3-Methyl-1,5-pentandiol, Neopentylglykol, alkoxylierte phenolische Verbindungen, wie ethoxylierte bzw. propoxylierte Bisphenole, 1,2-, 1,3- oder 1,4-Cyclohexandimethanol, trifunktionelle und höherfunktionelle Alkohole, wie Glycerin, Trimethylolpropan, Butantriol, Trimethylolethan, Pentaerythrit, Ditrimethylolpropan, Dipentaerythrit, Sorbit, Mannit und die entsprechenden alkoxylierten, insbesondere ethoxylierten und/oder propoxylierten Alkohole.

Die Alkoxylierungsprodukte sind in bekannter Weise durch Umsetzung der vorstehenden Alkohole mit Alkylenoxiden, insbesondere Ethylen- oder Propylenoxid, erhältlich. Vorzugsweise beträgt der Alkoxylierungsgrad je Hydroxylgruppe 0 bis 10, d.h. 1 mol Hydroxylgruppe kann mit bis zu 10 mol Alkylenoxiden alkoxyliert sein.

Als (Meth)acrylatverbindungen seien weiterhin Polyester(meth)acrylate genannt, wobei es sich um die (Meth)acrylsäureester oder Vinylether von Polyesterolen handelt, sowie Urethan-, Epoxid- oder Melamin(meth)acrylate.

Urethan(meth)acrylate sind z.B. erhältlich durch Umsetzung von Polyisocyanaten mit Hydroxyalkyl(meth)acrylaten und gegebenenfalls Kettenverlängerungsmitteln wie Diolen, Polyolen, Diaminen, Polyaminen oder Dithiolen oder Polythiolen.

Die Urethan(meth)acrylate haben vorzugsweise ein zahlenmittleres Molgewicht Mₙ von 500 bis 20 000, insbesondere von 750 bis 10 000 besonders bevorzugt 750 bis 3000 g/mol (bestimmt durch Gelpermeationschromatographie mit Polystyrol als Standard).

Die Urethan(meth)acrylate haben vorzugsweise einen Gehalt von 1 bis 5, besonders bevorzugt von 2 bis 4 Mol (Meth)acrylgruppen pro 1000 g Urethan(meth)acrylat.

Epoxid(meth)acrylate sind erhältlich durch Umsetzung von Epoxiden mit (Meth)acrylsäure. Als Epoxide in Betracht kommen z.B epoxidierte Olefine oder Glycidylether, z.B. Bisphenol-A-diglycidylether oder aliphatische Glycidylether, wie Butandioldiglycidether.

Melamin(meth)acrylate sind erhältlich durch Umsetzung von Melamin mit (Meth)acrylsäure oder deren Ester.

Die Epoxid(meth)acrylate und Melamin(meth)acrylate haben vorzugsweise ein zahlenmittleres Molgewicht Mₙ von 500 bis 20000, besonders bevorzugt von 750 bis 10000 g/mol und ganz besonders bevorzugt von 750 bis 3000 g/mol; der Gehalt an (Meth)acrylgruppen beträgt vorzugsweise 1 bis 5, besonders bevorzugt 2 bis 4 pro 1000 g Epoxid(meth)acrylat oder Melamin(meth)acrylat (bestimmt durch Gelpermeationschromatographie mit Polystyrol als Standard und Tetrahydrofuran als Elutionsmittel).

Weiterhin geeignet sind Carbonat(meth)acrylate, die im Mittel vorzugsweise 1 bis 5, insbesondere 2 bis 4, besonders bevorzugt 2 bis 3 (Meth)acrylgruppen und ganz besonders bevorzugt 2(Meth)acrylgruppen enthalten.

Das zahlungsmittlere Molekulargewicht Mₙ der Carbonat(meth)acrylate ist vorzugsweise kleiner 3000 g/mol, besonders bevorzugt kleiner 1500 g/mol, besonders bevorzugt kleiner 800 g/mol (bestimmt durch Gelpermeationschromatgraphie mit Polystyrol als Standard, Lösemittel Tetrahydrofuran).

Die Carbonat(meth)acrylate sind in einfacher Weise erhältlich durch Umesterung von Kohlensäureestern mit mehrwertigen, vorzugsweise zweiwertigen Alkoholen (Diolen, z.B. Hexandiol) und anschließende Veresterung der freien OH-Gruppen mit (Meth)-acrylsäure oder auch Umesterung mit (Meth)acrylsäureestern, wie es z.B. in EP-A 92 269 beschrieben ist. Erhältlich sind sie auch durch Umsetzung von Phosgen, Harnstoffderivaten mit mehrwertigen, z.B. zweiwertigen Alkoholen.

Als Reaktiwerdünner (Verbindungen (H)) kommen strahlungshärtbare, radikalisch oder kationisch polymerisierbare Verbindungen mit nur einer ethylenisch ungesättigten, copolymerisierbaren Gruppe in Betracht.

Genannt seien z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen, α,β-ungesättigte Carbonsäuren und deren Anhydride und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und 2-Ethylhexylacrylat. Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

α,β-Ungesättigte Carbonsäuren und deren Anhydride können beispielsweise sein Acrylsäure, Methacrylsäure, Fumarsäure, Crotonsäure, Itaconsäure, Maleinsäure oder Maleinsäureanhydrid, bevorzugt Acrylsäure.

Als vinylaromatische Verbindungen kommen z.B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar.

Als Photoinitiatoren (I) können dem Fachmann bekannte Photoinitiatoren, beispielsweise UV-Photoinitiatoren verwendet werden, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen z.B. Mono- oder Bisacylphosphinoxide Irgacure 819 (Bis(2,4,6-Trimethylbenzoyl)phenylphosphinoxid), wie sie z.B. in EP-A 7 508, EP-A 57 474, DE-A 196 18 720, EP-A 495 751 oder EP-A 615 980 beschrieben sind, beispielsweise 2,4,6-Trimethylbenzoyldiphenylphosphinoxid (Lucirin^{®} TPO), Ethyl-2,4,6-trimethylbenzoylphenylphosphinat, Benzophenone, Hydroxyacetophenone, Phenylglyoxylsäure und ihre Derivate oder Gemische dieser Photoinitiatoren. Als Beispiele seien genannt Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon, β-Methylanthrachinon, tert-Butylanthrachinon, Anthrachinoncarbonysäureester, Benzaldehyd, α-Tetralon, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon, Benzoin, Benzoin-iso-butylether, Chloroxanthenon, Benzoin-tetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Tri-o-Tolylphosphin, Benz[a]anthracen-7,12-dion, 2,2-Diethoxyacetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureestertyp, wie in DE-A 198 26 712, DE-A 199 13 353 oder WO 98/33761 beschrieben.

Unter den genannten Photoinitiatoren sind Phosphinoxide, α-Hydroxyketone und Benzophenone bevorzugt.

Insbesondere können auch Gemische verschiedener Photoinitiatoren verwendet werden.

Die Photoinitiatoren können allein oder in Kombination mit einem Photopolymerisationspromotor, z.B. vom Benzoesäure-, Amin- oder ähnlichem Typ verwendet werden.

Denkbar ist auch der Einsatz von IR-Photoinitiatoren.

IR-Photoinitiatoren enthalten ein Sensibilisator-Co-Inititiaror Gemisch. Als Sensibilisatorfarbstoff werden häufig Farbstoffe, insbesondere Cyanin-, Xanthylium- oder Thiazinfarbstoffe eingesetzt und als Co-Initiatoren beispielsweise Boranatsalze, Sulfoniumsalze, Iodoniumsalze, Sulfone, Peroxide, Pyridin-N-oxide oder Halogenmethyltriazine.

Als weitere lacktypische Additive (J) können beispielsweise Antioxidantien, Oxidationsinhibitoren, Stabilisatoren, Aktivatoren (Beschleuniger); Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Als Beschleuniger für die thermische Nachhärtung kann z.B. Zinnoctoat, Zinkoctoat, Dibutylzinnlaureat oder Diaza[2.2.2]bicyclooctan verwendet werden.

Weiterhin können ein oder mehrere photochemisch und/oder thermisch aktivierbare Initiatoren zugesetzt werden, z.B. Kaliumperoxodisulfat, Dibenzoylperoxid, Cyclohexanonperoxid, Di-tert.-Butylperoxid, Azobis-iso-butyronitril, Cyclohexylsulfonylacetylperoxid, Di-iso-propylpercarbonat, tert-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butylperoxid, Cumolhydroperoxid, Dicumylperoxid, t-Butylperbenzoat, silylierte Pinakole, die z. B. unter dem Handelsnamen AD-DID 600 der Firma Wacker kommerziell erhältlich sind oder Hydroxylgruppen-haltige Amin-N-Oxide, wie 2,2,6,6-Tetramethylpiperidin-N-oxyl, 4-Hydroxy-2,2,6,6-Tetramethylpiperidin-N-oxyl etc.

Weitere Beispiele geeigneter thermisch aktivierbarer Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonite in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil^{®} der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin^{®} -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Weiterhin geeignete Stabilisatoren sind beispielsweise N-Oxyle, wie z.B. 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, Phenole und Naphthole, wie z.B. p-Aminophenol, p-Nitrosophenol, 2-tert.-Butylphenol, 4-tert.-Butylphenol, 2,4-di-tert.-Butylphenol, 2-Methyl-4-tert.-Butylphenol, 4-Methyl-2,6-tert.-Butylphenol (2,6-tert.-Butyl-p-Kresol) oder 4-tert.-Butyl-2,6-dimethylphenol, Chinone, wie z.B. Hydrochinon oder Hydrochinonmonomethylether, aromatische Amine, wie z.B. N,N-Diphenylamin, N-Nitroso-diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoff atome bestehen und geradkettig oder verzweigt sein können, Hydroxylamine, wie z.B. N,N-Diethylhydroxylamin, Harnstoffderivate, wie z.B. Harnstoff oder Thioharnstoff, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin.

Typische Zusammensetzungen für strahlungshärtbare Massen sind beispielsweise
(F) 20 -100 Gew%, bevorzugt 40 - 90, besonders bevorzugt 50 - 90 und insbesondere 60 - 80 Gew%,
(G) 0-60 Gew%, bevorzugt 5 - 50, besonders bevorzugt 10-40 und insbesondere 10-30 Gew%,
(H) 0-50 Gew%, bevorzugt 5 - 40, besonders bevorzugt 6-30 und insbesondere 10-30 Gew%,
(I) 0-20 Gew%, bevorzugt 0,5 - 15, besonders bevorzugt 1-10 und insbesondere 2-5 Gew% sowie
(J) 0 - 50 Gew%, bevorzugt 2 - 40, besonders bevorzugt 3-30 und insbesondere 5-20 Gew%,
mit der Maßgabe, daß (F), (G), (H), (I) und (J) zusammen 100 Gew% ergeben.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Bechichtungsmasse, gegebenenfalls unter Erhitzen, entfernt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man die Beschichtungsmasse auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur.

Die Trocknung kann auch zusätzlich oder anstelle der thermischen Trocknung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.
Gegebenenfalls kann, wenn mehrere Schichten des Beschichtungsmittels übereinander aufgetragen werden, nach jedem Beschichtungsvorgang eine thermische und/oder NIR-Trocknung und Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ= 200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier.
Diese können auch in jeweils unterschiedlichen Wellenlängebereichen strahlen.

Die Bestrahlung kann gegebenenfalls auch unter Ausschluß von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Desweiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.

Weiterhin sind auch Substrat, beschichtet mit einer Mehrschichtlackierung Gegenstand der vorliegenden Anmeldung.

Die Dicke einer solche wie beschrieben zu härtenden Schicht kann von 0,1 µm bis mehrere mm betragen, bevorzugt von 1 bis 2000 µm, besonders bevorzugt 5 bis 1000 µm, ganz besonders bevorzugt von 10 bis 500 µm und insbesondere von 10 bis 250 µm.

Die erfindungsgemäß hergestellten (Meth)acrylsäureester können aufgrund ihrer geringeren Färbung vorteilhaft auch in einer thermisch induzierten (radikalischen) (Co)polymerisation eingesetzt werden.

Als Monomere, mit denen die erfindungsgemäß hergestellten (Meth)acrylsäureester beispielsweise copolymerisiert werden können seien genannt z.B. C₁-C₂₀-Alkyl(meth)-acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und verzweigte Alkylderivate wie 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

Als vinylaromatische Verbindungen kommen z.B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Eine häufige, aber nicht die einzige Methode zur Herstellung solcher (Co)Polymerisate ist die radikalische oder ionische (Co)Polymerisation in einem Lösungs- oder Verdünnungsmittel.

Die radikalische (Co)Polymerisation solcher Monomere erfolgt beispielsweise in wäßriger Lösung in Gegenwart von Polymerisationsinitiatoren, die unter Polymerisationsbedingungen in Radikale zerfallen, beispielsweise Peroxodisulfate, H₂O₂-Redoxysysteme oder Hydroxyperoxide, wie z.B. tert. Butylhydroperoxid oder Cumolhydroperoxid. Die (Co)Polymerisation kann in einem weiten Temperaturbereich, gegebenenfalls unter vermindertem oder auch unter erhöhtem Druck in der Regel bei Temperaturen bis zu 100 °C vorgenommen werden. Der pH-Wert des Reaktionsgemisches wird gewöhnlich in dem Bereich von 4 bis 10 eingestellt.

Die (Co)Polymerisation kann aber auch in anderer, dem Fachmann an sich bekannter Weise kontinuierlich oder diskontinuierlich durchgeführt werden, z.B. als Lösungs-, Fällungs-, Wasser-in-Öl-Emulsions-, inverse Emulsions, Suspensions oder umgekehrte Suspensionspolymerisation.

Dabei wird das Monomer/die Monomere unter Verwendung radikalischer Polymerisationsinitiatoren, z.B. in Radikale zerfallende Azoverbindungen, wie 2,2'-Azo-bis(iso-butyronitril), 2,2'-Azobis-(2-amidinopropan)-hydrochlorid oder 4,4'-Azo-bis-(4'-cyanpentansäure) oder Dialkylperoxide, wie Di-tert.-Amylperoxid, Aryl-alkylperoxide, wie tert.-Butyl-cumylperoxid, Alkyl-acylperoxide, wie tert.-Butyl-peroxy-2-ethylhexanoat, Peroxidicarbonate, wie Di-(4-tert.-Butylcyclohexyl)peroxydicarbonat oder Hydroperoxide (co)polymerisiert.

Die genannten Verbindungen werden meist in Form wäßriger Lösungen oder wäßriger Emulsionen eingesetzt, wobei die untere Konzentration durch die in der (Co)Polymerisation vertretbare Wassermenge und die obere Konzentration durch die Löslichkeit der betreffenden Verbindung in Wasser bestimmt ist.

Als Lösungs- oder Verdünnungsmittel können dienen z.B. Wasser, Alkohole, wie Methanol, Ethanol, n- oder iso-Propanol, n- oder iso-Butanol, oder Ketone, wie Aceton, Ethylmethylketon, Diethylketon oder iso-Butylmethylketon. Besonders bevorzugt sind unpolare Lösungsmittel wie beispielsweise Xylol und dessen Isomerengemische, Shellsol® A und Solventnaphtha.

In einer bevorzugten Ausführungsform werden die Monomere vorgemischt und Initiator mit gegebenenfalls weiteren Zusätzen gelöst in Lösungsmittel zugegeben. Eine besonders bevorzugte Ausführungsform ist beschrieben in WO 01/23484 und dort besonders auf Seite 10, Z. 3 bis Z. 24

Gegebenenfalls kann die (Co)Polymerisation in Gegenwart von Polymerisationsreglern, wie beispielsweise Hydroxylammoniumsalze, chlorierte Kohlenwasserstoffe und Thioverbindungen, wie z.B. tert.-Butylmercaptan, Thioglycolsäureethylacrylester, Mercaptoethynol, Mercaptopropyltrimethoxysilan, Dodecylmercaptan, tert.-Dodecylmercaptan oder Alkalimetallhypophosphite, durchgeführt werden. Bei der (Co)Polymerisation können diese Regler, z. B. in Mengen von 0 bis 0,8 Gew.-Teile, bezogen auf 100 Gew.-Teile der zu (co)polymerisierenden Monomeren, eingesetzt werden, durch die die Molmasse des entstehenden (Co)Polymers verringert wird.

Bei der Emulsionspolymerisation können Dispergiermittel, ionische und/oder nichtionische Emulgatoren und/oder Schutzkolloide bzw. Stabilisatoren als grenzflächenaktive Verbindungen verwendet werden.
Als solche kommen sowohl die zur Durchführung von Emulsionspolymerisationen üblicherweise eingesetzten Schutzkolloide als auch Emulgatoren in Betracht.

Geeignete Schutzkolloide sind beispielsweise Polyvinylalkohole, Cellulosederivate oder Vinylpyrrolidon enthaltende Copolymerisate. Eine ausführliche Beschreibung weiterer geeigneter Schutzkolloide findet sich in Houben-Weyl, Methoden der organischen Chemie, Band XIV/1, makromolekulare Stoffe, Georg-Thieme-Verlag, Stuttgart, 1969, S. 411 bis 420. Selbstverständlich können auch Gemische aus Emulgatoren und/oder Schutzkolloiden verwendet werden. Vorzugsweise werden als Dispergiermittel ausschließlich Emulgatoren eingesetzt, deren relative Molekulargewichte im Unterschied zu den Schutzkolloiden üblicherweise unter 1000 liegen. Sie können sowohl anionischer, kationischer oder nichtionischer Natur sein. Selbstverständlich müssen im Falle der Verwendung von Gemischen granzflächenaktiver Substanzen die Einzelkomponenten miteinander verträglich sein, was im Zweifelsfall an Hand weniger Vorversuche überprüft werden kann. Im allgemeinen sind anionische Emulgatoren untereinander und mit nichtionischen Emulgatoren verträglich.

Desgleichen gilt auch für kationische Emulgatoren, während anionische und kationische Emulgatoren meistens miteinander unverträglich sind. Gebräuchliche Emulgatoren sind z.B. ethoxylierte Mono-, Di- und Tri-Alkylphenole (EO-Grad: 3 bis 100, : C₄ bis C₁₂), ethoxylierte Fettalkohole (EO-Grad: 3 bis 100, Alkylrest: C₈ bis C₁₈), sowie Alkali- und Ammoniumsalze von Alkylsulfaten (Alkylrest: C₈ bis C₁₆) von Schwefelsäurehalbestern ethoxylierter Alkylphenole (EO-Grad: 3 bis 100, Alkylrest: C₄ bis C₁₂), von Alkylsulfonsäuren (Alkylrest: C₁₂ bis C₁₈) und von Alkylacrylsulfonsäuren (Alkylrest: C₉ bis C₁₈). Weitere geeignete Emulgatoren wie Sulfobernsteinsäureester finden sich in Houben-Weyl, Methoden der organischen Chemie, Band XIV/1, Makromolekulare Stoffe, Georg-Thieme Verlag, Stuttgart, 1961, Seiten 192 bis 208.

In der Regel beträgt die Menge an eingesetzten Dispergiermittel 0,5 bis 6, vorzugsweise 1 bis 3 Gew.-% bezogen auf die radikalisch zu polymerisierenden Monomeren.

Beispiele für (meth)acrylathaltige Dispersionen sind n-Butylacrylat/Acrylnitril - Dispersionen, die als Klebstoffe Anwendung finden, n-Butylacrylat/Butadien/Styrol-Die Polymerdispersionen, in denen erfindungsgemäß hergestellte (Meth)acrylsäureester verwendet werden, können zusätzlich chemisch und/oder physikalisch desodoriert werden.

Die mit den erfindungsgemäß hergestellten (Meth)acrylsäureestern erhältlichen Copolymerisate weisen in der Regel eine geringere Farbzahl auf, was im Lackbereich vorteilhaft ist. Die beschriebenen Copolymerisate lassen sich dann in an sich bekannter Weise beispielsweise mit Aminoplasten, wie z.B. Melamin, zu vernetzten Lackharzen umsetzen, wie es beispielsweise beschrieben ist in der EP 738740 oder EP 675141.

Besonders bevorzugt eignen sich die Beschichtungsmassen als oder in Außenbeschichtungen, also solche Anwendungen, die dem Tageslicht ausgesetzt sind, bevorzugt von Gebäuden oder Gebäudeteilen, Innenbeschichtungen, Straßenmarkierungen, Beschichtungen auf Fahrzeugen und Flugzeugen. Insbesondere werden die Beschichtungen als Holz-, Papier- oder Kunststoffbeschichtungen, beispielsweise für Parkett oder Möbel eingesetzt.

Ein weiterer Gegenstand ist die Verwendung der erfindungsgemäß erhaltenen Produkte als Vorprodukt für Glanzzusätze in der Galvanotechnik. Durch ihre gegenüber konventionell erhältlichen Produkten verringerte Farbzahl macht sie für diese Anwendung außerordentlich geeignet.

Mit Hilfe des erfindungsgemäßen Verfahrens ist die Herstellung (Meth)acrylsäureester (F) in hoher chemischer und Raum-Zeit-Ausbeute und unter milden Bedingungen mit guten Farbzahlen möglich. Trotz Verzicht auf aktivierte (Meth)Acrylsäureverbindungen werden gezielt die gewünschten Produkte mit hoher Selektivität erhalten, die weitgehend frei von Nebenprodukten sind.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

Golpanol® PME ist ein kommerziell erhältliches Produkt der BASF AG, Ludwigshafen. Dabei handelt es sich im ein Gemisch aus ein- und zweifach ethoxyliertem Propargylalkohol.

### Beispiel 1

Umesterung von Golpanol® PME mit unterschiedlichen Überschüssen Methylacrylat

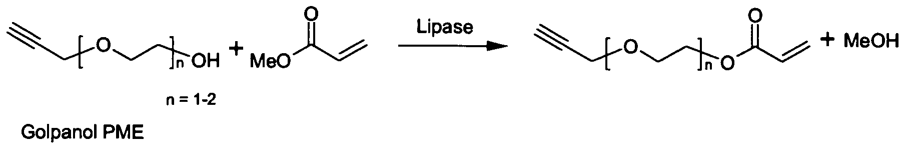

Golpanol® PME (5 mmol, 501 mg) wurden mit Methylacrylat, 25 mg Novozym® 435 (Lipase B aus Candida antartica) und 1,0 g Molsieb 5 Å für 24 h bei 40 °C geschüttelt und der Umsatz mittels Gaschromatographie bestimmt.

| Menge Methylacrylat [mmol] | Umsatz [%] |
|---|---|
| 50 | 100 |
| 40 | 100 |
| 30 | 100 |
| 20 | 99 |
| 20 (ohne Enzym) | 10 |
| 10 | 92 |

### Beispiel 2

### Präparativer Ansatz mit Golpanol PME

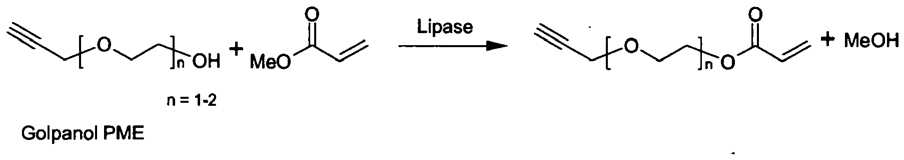

In einem 4L-Rundkolben mit aufgesetztem Rückflusskühler wurden 1377 g Methylacrylat (16,0 mol), 400,5 g Golpanol® PME (4,0 mol, Farbzahl von 47 nach Hazen (APHA)), 800 g Molekularsieb 5 Å und 50 mg Hydrochinonmonomethylether vorgelegt und die Reaktion durch Zugabe von 20,0 g Novozym® 435 gestartet. Nach 7 h Rühren bei 40 °C wurden die Feststoffe über eine Filternutsche entfernt. Der Überschuss Methylacrylat wurde am Rotationsverdampfer (40 °C, 6 mbar) entfernt. Laut gaschromatographischer Analyse war der Alkohol zu 99,5 % verestert. Man erhielt 557 g (93 % Ausbeute) 2-Propargyloxethylacrylat als klare, leicht gelbliche Flüssigkeit mit einer Farbzahl von 79 nach Hazen (APHA, gemäß DIN ISO 6271).

### Beispiel 3

### Umsetzung von Propargylalkohol

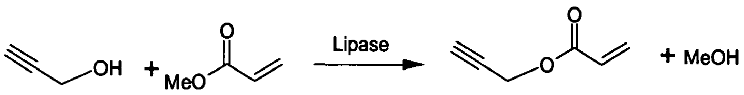

Propargylalkohol (5 mmol, 295 µl) wurden mit 50 mmol Methylacrylat (4,51 ml), 25 mg Novozym® 435 und evtl. 1,0 g Molsieb 5 Å für 24 h bei 40 °C geschüttelt und der Umsatz mittels GC bestimmt.

| Molsieb Zugabe | Umsatz [%] |
|---|---|
| ohne | 58 |
| mit | 95 |

## Patentansprüche

1. Verfahren zur Herstellung von (Meth)acrylsäureestern (F) von mindestens eine C-C-Dreifachbindung aufweisenden Alkoholen, **dadurch gekennzeichnet, daß** man mindestens einen mindestens eine C-C-Dreifachbindung aufweisenden Alkohol (C) der ausgewählt ist aus Propargylalkohol oder dessen ein- oder zweifach ethoxylierter Produkte sowie Gemische daraus,
in Gegenwart mindestens eines Enzyms (E) mit mindestens einem (Meth)acrylsäureester (D) umestert,
wobei der (Meth)acrylsäureester (D) ein gesättigter C₁ - C₁₀-Alkylester oder C₃ C₁₂-Cyclo-alkylester der (Meth)acrylsäure ist,
wobei das Enzym (E) eine Lipase B aus Candida antarctica ist, und
wobei das molare Verhältnis von (Meth)acrylsäureverbindung (D) (bezogen auf die (Meth)acryleinheiten) zu Alkohol (C) (bezogen auf Hydroxygruppen) im Bereich von 20:1 bis 2:1 liegt.

2. Verfahren nach einem der vorstehenden Ansprüche, **dadurch gekennzeichnet, daß** Verbindungen (D) ein C₁ - C₄-Alkylester der (Meth)acrylsäure ist.

3. Verfahren nach einem der vorstehenden Ansprüche, **dadurch gekennzeichnet, daß** das molare Verhältnis von (Meth)acrylsäureverbindung (D) (bezogen auf die (Meth)acryleinheiten) zu Alkohol (C) (bezogen auf Hydroxygruppen) im Bereich von 20:1 bis 4:1 liegt.

## Claims

1. A process for preparing a (meth)acrylic ester (F) of an alcohol having at least one C-C triple bond, which comprises subjecting at least one alcohol (C) having at least one C-C tripe bond, selected from propargyl alcohol or its mono- or di- ethoxylated products, and also mixtures thereof, to transesterification with at least one (meth) acrylic ester (D) in the presence of at least one enzyme (E),
the (meth)acrylic ester (D) being a saturated C₁-C₁₀ alkyl ester or C₃-C₁₂ cycloalkyl ester of (meth)acrylic acid,
the enzyme (E) being a lipase B from Candida antarctica, and
the molar ratio of (meth)acrylic acid compound (D) (based on the (meth)acrylic units) to alcohol (C) (based on hydroxy groups) being in the range from 20:1 to 2:1.

2. The process according to claim 1, wherein compound (D) is a C₁-C₄ alkyl ester of (meth)acrylic acid.

3. The process according to either of the preceding claims, wherein the molar ratio of (meth)acrylic acid compound (D) (based on the (meth)acrylic units) to alcohol (C) (based on hydroxyl groups) is in the range from 20:1 to 4:1.

## Revendications

1. Procédé de préparation d'esters de l'acide (méth)acrylique (F) d'alcools présentant au moins une triple liaison C-C, **caractérisé en ce qu'**on transestérifie au moins un alcool (C) présentant au moins une triple liaison C-C, qui est choisi parmi l'alcool propargylique ou ses produits monoéthoxylés ou diéthoxylés ainsi que leurs mélanges, en présence d'au moins une enzyme (E) avec au moins un ester de l'acide (méth)acrylique (D),
l'ester de l'acide (méth)acrylique (D) étant un ester C₁-C₁₀-alkylique saturé ou C₃-C₁₂-cycloalkylique saturé de l'acide (méth)acrylique,
l'enzyme (E) étant une lipase B de Candida antarctica, et
le rapport molaire du composé d'acide (méth)acrylique (D) (par rapport aux unités (méth)acryle) à l'alcool (C) (par rapport aux groupes hydroxy) se situant dans la plage de 20:1 à 2:1.

2. Procédé selon la revendication précédente, **caractérisé en ce que** le composé (D) est un ester C₁-C₄-alkylique de l'acide (méth) acrylique.

3. Procédé selon l'une quelconque des revendications précédentes, **caractérisé en ce que** le rapport molaire du composé d'acicle (méth)acrylique (D) (par rapport aux unités (méth)acryle) à l'alcool (C) (par rapport aux groupes hydroxy) se situe dans la plage de 20:1 à 4:1.
